# EUROPEAN PATENT SPECIFICATION

(11) **EP 3 027 063 B1**
(45) Date of publication and mention of the grant of the patent: **21.11.2018**
(21) Application number: 14747618.8
(22) Date of filing: 31.07.2014
(51) Int. Cl.: A23L 33/10, A23L 33/00, A61K 31/20, A61K 31/23, A61P 25/28

(54) **OPO GLYCERIDE COMPOSITION**
OPO-GLYCERIDZUSAMMENSETZUNG
COMPOSITION OPO-GLYCÉRIDIQUE

(30) Priority: 01.08.2013 EP 13275180
(43) Date of publication of application: 08.06.2016
(73) Proprietor: Bunge Loders Croklaan B.V., 1521 AZ Wormerveer (NL)
(72) Inventor: GAMBELLI, Luisa, NL-1521 AZ Wormerveer (NL); HAPPE, Randolph, NL-1521 AZ Wormerveer (NL); BANNI, Sebastiano, I-09126 Cagliari (IT)
(74) Representative: Potter Clarkson LLP
(86) International application number: PCT/EP2014/066549
(87) International publication number: WO 2015/014967

(56) References cited:
- WO-A1-2005/037373
- WO-A1-2005/051091
- WO-A1-2007/029018
- WO-A1-2009/057121
- CARLA TECELÃO ET AL: "Production of human milk fat substitutes enriched in omega-3 polyunsaturated fatty acids using immobilized commercial lipases and Candida parapsilosis lipase/acyltransferase", JOURNAL OF MOLECULAR CATALYSIS. B, ENZYMATIC, ELSEVIER, AMSTERDAM, NL, vol. 65, no. 1-4, 1 August 2010 (2010-08-01), pages 122-127, XP002687978, ISSN: 1381-1177, DOI: 10.1016/J.MOLCATB.2010.01.026 [retrieved on 2010-02-01]
- NE E AHÍN ET AL: "Human Milk Fat Substitutes Containing Omega-3 Fatty Acids", JOURNAL OF AGRICULTURAL AND FOOD CHEMISTRY, AMERICAN CHEMICAL SOCIETY, US, vol. 54, no. 10, 17 May 2006 (2006-05-17), pages 3717-3722, XP002687979, ISSN: 0021-8561, DOI: 10.1021/JF053103F [retrieved on 2006-04-12]
- TABATA H ET AL: "Incorporation of plasma palmitate into the brain of the rat during development", DEVELOPMENTAL BRAIN RESEARCH, ELSEVIER SCIENCE BV, AMSTERDAM, NL, vol. 29, no. 1, 1 September 1986 (1986-09-01), pages 1-8, XP024532253, ISSN: 0165-3806, DOI: 10.1016/0165-3806(86)90076-3 [retrieved on 1986-09-01]
- Sheila M. Innis ET AL: "Dietary triacyglycerols rich in sn-2 palmitate alter post-prandial lipoprotein and unesterified fatty acids in term infants", PROSTAGLANDINS LEUKOTRIENES AND ESSENTIAL FATTY ACIDS., vol. 89, no. 4, 1 September 2013 (2013-09-01), pages 145-151, XP055287108, EDINBURGH ISSN: 0952-3278, DOI: 10.1016/j.plefa.2013.03.003
- Fabiana Bar-Yoseph ET AL: "Review of sn-2 palmitate oil implications for infant health", PROSTAGLANDINS LEUKOTRIENES AND ESSENTIAL FATTY ACIDS., vol. 89, no. 4, 1 September 2013 (2013-09-01), pages 139-143, XP055238040, EDINBURGH ISSN: 0952-3278, DOI: 10.1016/j.plefa.2013.03.002
- D.J Sanders ET AL: "The absorption, distribution and excretion of 1- and 2-[14C]palmitoyl triacyglycerols in the rat", FOOD AND CHEMICAL TOXICOLOGY., vol. 39, no. 7, 1 July 2001 (2001-07-01), pages 709-716, XP055377411, GB ISSN: 0278-6915, DOI: 10.1016/S0278-6915(01)00017-5

## Description

This invention relates to a composition for use in developing and/or improving brain function, particularly in infants.

Triglyceride fats and oils are important commercial products and are used extensively in, for example, the food industry. Some triglycerides are nutritionally important and the triglyceride 1,3-dioleoyl-2-palmitoyl glyceride (OPO) is known to be an important component of human milk fat.

Fat compositions containing the principal fatty acids found in human milk fat, in similar amounts to those found in human milk fat, may be derived from oils and fats of vegetable origin. However, a significant difference in composition arises because most glycerides of vegetable origin are unsaturated in the 2-position. In contrast, a substantial amount of palmitic acid occupies the 2-position of glycerides in human milk fat.

Innis, Adv. Nutr., 2:275-283, 2011 describes the role of dietary triacylglycerol structure in infant nutrition.

Koletzko et al, Journal of Pediatric Gastroenterology and Nutrition, 41:584-589, November 2005, describes a recommended composition for infant formula.

Processes are known for selectively producing the triglyceride 1,3-dioleoyl-2-palmitoyl glyceride (OPO) for use in replacements for human milk fat.

EP-A-0209327 discloses milk replacement fat compositions comprising the triglyceride 1,3-dioleoyl-2-palmitoyl glyceride (OPO). The fat compositions can be obtained by subjecting fatty mixtures of 2-palmitoyl glycerides to reaction with oleic acid in the presence of a catalyst, such as a lipase, which is regiospecific in activity in the 1- and 3-positions of the glycerides. Enzymatic processes of this kind are also described in GB-A-1577933. Under the influence of the catalyst, unsaturated fatty acid residues may be introduced into the 1- and 3- positions of the 2-palmitoyl glycerides by exchange with unsaturated free fatty acids or their alkyl esters.

WO 2005/036987 discloses a process for producing a fat base by reacting a palmitic rich oil with unsaturated fatty acids such as oleic acid. The total palmitic acid residue content of the fat base is at most 38% and at least 60% of the fatty acid moieties are in the 2-position of the glyceride backbone. A related disclosure can be found in WO 2005/037373, filed on the same day.

WO 2007/029018 describes a process for the production of a composition comprising 1,3-dioleoyl-2-palmitoyl glyceride (OPO) in which a palm oil stearin, with an iodine value (IV) between about 2 and about 12 is subjected to enzymatic transesterification, with oleic acid or a non-glyceride ester of oleic acid.

Docosahexaenoic acid (DHA) is an important fatty acid that is required for the development of the brain. It is particularly important in infants to maintain the correct DHA level in the brain.

Innis, J. Nutr. 137:855-859, 2007 relates to dietary (n-3) fatty acids, particularly docosahexaenoic acid (DHA), and their role in brain development.

Delplanque et al, Prostaglandins, Leukotrienes and Essential Fatty Acids, 2012, http://dx.doi.org/10.1016/j.plefa.2012.07.004 discloses that a dairy fat matrix providing alpha-linolenic acid (ALA) is better than a vegetable fat mixture for increasing brain DHA accretion in young rats.

It is recognised that dietary fat may influence brain function and neuroinflammation. A role of the peroxisome proliferator-activated receptor PPAR-alpha has been recognised in several brain areas as a player in controlling pro-inflammatory mediators and in modulating neurotransmitter release. PPAR-alpha induces peroxisomal beta-oxidation, which is one of the major catabolic pathways of pro-inflammatory eicosanoids, and also induces the formation of secondary signalling molecules, which modulate neurotransmitter releases.

One of the major documented endogenous ligands of PPAR-alpha in the brain is oleoylethanolamide (OEA), a congener molecule of anandamide (AEA), which shares with OEA the same biosynthetic and degradation pathways.

Campolongo et al, Proceedings of the National Academy of Sciences, 2009, 106(19), 8027-8031 discloses that administration of oleoylethanolamide enhances memory consolidation.

Mazzola, et al, Learning & Memory, 2009, 16(5), 332-337, discloses the effect of activation of PPAR-alpha receptors on memory enhancement by showing that memory acquisition can acutely be enhanced through actions involving PPAR-alpha ligand

Moreno, et al, Neuroscience, 2004, 123, 131-145, discloses that PPAR-alpha receptors are present in relatively large numbers in memory related brain areas.

There remains a need for compositions that can develop and/or improve brain function, particularly in infants. In particular, there is a need for compositions that can develop and/or improve brain function whilst already being recognised as being safe for food use.

According to the invention, there is provided 1,3-dioleoyl-2-palmitoyl glyceride (OPO) for use in developing and/or improving brain function, protecting against neuroinflammation or treating and/or ameliorating addiction or Alzheimer's disease.

The invention relates to developing and/or improving brain function. Preferably, the invention involves the development of brain function in infant humans (typically from 0 to 4 years old). However, the invention may involve developing and/or improving brain function in infants and older humans. Brain function that may be developed and/or improved according to the invention preferably includes, for example, one or more of motor skills, learning, memory, intelligence (for example IQ), language skills and cognitive functions.

The invention involves a surprising increase in oleoylethanolamide (OEA) levels in the brain caused by the glyceride or the composition comprising the glyceride. This may exert several beneficial effects, in view of the known effects of OEA in the brain, in developing and/or improving brain function, such as protection from neuroinflammation, modulation of the dopaminergic system and improvement of memory. Without wishing to be bound by theory, it is believed that palmitic acid at the 2- position in dietary triglycerides triggers an increase of OEA biosynthesis, which may contribute to the physiological brain function and development, for example in infants.

The term glyceride, as used herein, includes mono-, di- and tri- glycerides of fatty acids, and mixtures thereof, unless otherwise specified.

The term fatty acid as used herein refers to saturated or unsaturated, straight chain carboxylic acids having from 6 to 24 carbon atoms, preferably from 12 to 22 carbon atoms. Unsaturated acids may comprise one, two, or more double bonds, preferably one or two double bonds.

The glyceride used in the invention is preferably derived from vegetable sources, more preferably from palm oil. The glyceride is typically synthesised from palm oil or a fraction of palm oil. It will therefore be appreciated that the term derived from vegetable sources means that the glyceride is obtained directly or indirectly from vegetable sources and that the glyceride is not obtained directly or indirectly from animal products.

The glyceride used in the invention is 1,3- dioleoyl-2-palmitoyl glyceride (OPO). The OPO content of the composition is preferably from 1 % to 90 % by weight, more preferably from 5 % to 75 % by weight, even more preferably from 10 % to 50 % by weight, such as from 10 % to 30 % by weight.

The composition comprising the glyceride having a palmitoyl group at the 2- position has at least 40% by weight of the palmitic acid content of the composition present as palmitoyl groups bonded at the 2- position of a glyceride. A preferred range for the palmitic acid content of the composition present as palmitoyl groups bonded at the 2-position of a glyceride is from 40% to 60% by weight. The percentage of palmitic acid residues in the 2- position is preferably determined by finding: (a) the total palmitic content of the fat by FAME (fatty acid methyl ester analysis) (surface internal standard C17:0); and (b) the palmitic content of the 2- position by FAME (same standard) of a sample of the fat after hydrolysis of the 1- and 3- residues using pancreatic lipase to form a 2-monoglyceride. The value is ((b) multiplied by 100) ÷ ((a) multiplied by 3).

In one aspect of the invention, at least 45%, at least 50%, at least 60% or at least 70% by weight of the palmitic acid content of the composition is present as palmitoyl groups bonded at the 2- position of a glyceride.

Preferably, the composition is free of phospholipids.

Optionally, the composition of the invention comprises alpha-linolenic acid (ALA) or a derivative thereof in an amount of greater than 1% by weight alpha-linolenic acid (in free and bound forms) based on total free and bound C₆ to C₂₄ fatty acids. It is believed that the alpha-linolenic acid or derivative may co-operate with the glyceride in the invention to assist in developing and/or improving brain function. More preferably, the amount of alpha-linolenic acid based on total free and bound C₆ to C₂₄ fatty acids in the composition of the invention is greater than 1.3%, such as from 1.3% to 2.0%, even more preferably greater than 1.5% by weight.

Derivatives of alpha-linolenic acid (ALA) or other fatty acids include non-toxic salts and esters, such as sodium salts, C₁ to C₆ alkyl esters (preferably straight chain) and mono-, di- and tri- glycerides. Glycerides are preferred derivatives.

The composition of the invention may comprise linoleic acid or a glyceride thereof. The weight ratio of total free and bound linoleic acid to total free and bound alpha-linolenic acid is preferably 15:1 or less, more preferably from 15:1 to 5:1. The amount of linoleic acid or glyceride thereof in the composition of the invention is preferably from 12 to 14% by weight based on total free and bound C₆ to C₂₄ fatty acids in the composition.

The terms free and bound refer to fatty acids that are either free carboxylic acids or their salts, or are covalently bonded to another group, for example as esters such as glycerides. For example, the total free and bound fatty acid (such as alpha-linolenic acid) in a glyceride composition includes the fatty acid present in mono-, di- and triglycerides. The total free and bound fatty acid content of a composition may be determined by FAME analysis.

The composition may be a blend comprising (i) a composition comprising alpha-linolenic acid or a glyceride thereof and (ii) a triglyceride composition comprising OPO in an amount of at least 20% by weight. The composition comprising alpha-linolenic acid or a glyceride thereof is preferably a vegetable oil. Preferred sources of alpha-linolenic acid are canola (rapeseed) oil, flaxseed oil and mixtures thereof.

The composition of the invention is preferably a blend of (a) a triglyceride composition comprising OPO and having at least 70% by weight of the palmitic acid content of the composition present as palmitoyl groups bonded at the 2- position and (b) one or more liquid oils, more preferably a mixture of canola (rapeseed) oil, sunflower oil and palm kernel oil. Preferably, the blend comprises 30-60% by weight of (a) and 40-70% by weight of (b).

The triglyceride composition comprising OPO may be produced by a process comprising the reaction of palm oil stearin with oleic acid or an ester thereof in the presence of a lipase.

For example, the triglyceride composition comprising OPO may be produced by a process which comprises:
(i) providing a palm oil stearin comprising tripalmitoyl glyceride and having an iodine value of between about 2 and about 14;
(ii) optionally bleaching and deodorising the palm oil stearin;
(iii) subjecting the palm oil stearin to enzymic transesterification with oleic acid or a non-glyceride ester thereof;
(iv) separating palmitic acid or palmitic non-glyceride esters from the product obtained in (iii); and
(v) optionally dry fractionating the product obtained in (iv) to form a fraction comprising an increased amount of OPO.

The palm oil stearin used in step (i) of the process may be obtained from solvent (wet) fractionation, Lanza fractionation, or dry fractionation of palm oil, such as multi-stage counter current dry fractionation of palm oil. The palm oil can be crude palm oil, refined palm oil, fractions of palm oil (e.g., obtained by dry fractionation), other derivatives of palm oil, or mixtures thereof. The palm oil stearin preferably has a palmitic acid content of at least 60% by weight, more preferably at least 70% by weight, such as at least 75%, at least 80% or at least 85% by weight e.g., at least 90% by weight, based on the total fatty acid residue content. The palm oil stearin used in step (i), preferably comprises 2-palmitoyl glycerides, typically in an amount of greater than 50% by weight, such as greater than 55% by weight or greater than 60% by weight. The palm oil stearin preferably has an iodine value (IV) of between about 2 and about 14, preferably between about 4 and about 14, more preferably between about 6 and about 12, such as from about 8 to about 12. The palm oil stearin is optionally randomly interesterified before or after (ii).

Before the palm oil stearin is subjected to enzymatic esterification, the palm oil stearin is typically refined in optional step (ii), which preferably involves bleaching and deodorising. The bleaching of the palm oil stearin in the process is performed above 95°C, more preferably above 100°C (such as at from 105°C to 120°C). In the deodorising step, volatile impurities are removed from the palm oil stearin to yield deodorised palm oil stearin, typically at temperatures above 200°C. The impurities removed in the deodorising step commonly include free fatty acids, aldehydes, ketones, alcohols and other hydrocarbon impurities. The bleaching and deodorising are performed under standard conditions known in the art and may be carried out in a single process step or two or more process steps. For example, the steps may be carried out at reduced pressures (e.g., 10 mm Hg or below), wherein the palm oil stearin is contacted with steam to help vaporise the impurities. Bleaching and deodorising the palm oil stearin may help to improve the yield of the process.

The enzymic transesterification according to the process is preferably carried out using a 1,3 specific lipase as a biocatalyst.

Under the influence of a 1,3 lipase, unsaturated fatty acid residues may be introduced into the 1- and 3- positions of the 2-palmitoyl glycerides by exchange with the fatty acid residues of other glycerides or more preferably by means of transesterification in the fatty mixture. Exchange preferably takes place between unsaturated free fatty acids, preferably oleic acid, or alkyl esters of oleic acid with alcohols having from 1 to 6 carbon atoms. The 2-palmitoyl glycerides modified in this way may be separated from the reaction mixture.

The enzymic transesterification reaction selectively exchanges palmitic acid with oleic acid on the 1,3-position rather than the 2-position. The transesterification reaction is typically performed to reach or approach equilibrium at a conversion ratio of a minimum of at least 50%, preferably at least 60%, most preferably at least 70%.

Preferably, in the transesterification reaction, the palm oil stearin is, for example, mixed with an oleic acid concentrate (comprising free oleic acid at a concentration of greater than 65% by weight, preferably greater than 70% by weight, most preferably greater than 75% by weight). Alternatively, the oleic acid may be provided as a mixture comprising oleic acid (preferably in an amount of greater than 65% by weight), linoleic acid and, optionally, one or more other fatty acids. The ratio of palm oil stearin to oleic acid concentrate is preferably from 0.1:1 to 2:1, more preferably from 0.4:1 to 1.2:1, even more preferably from 0.4:1 to 1:1, most preferably from 1:1.1 to 1:2 on a weight basis. The reaction is preferably carried out at a temperature of from 30 °C to 90 °C, preferably from 50 °C to 80 °C, such as about 60 °C to 70 °C, and may be conducted batchwise or in continuous fashion, with or without a water-immiscible organic solvent.

Before the enzyme transesterification reaction, the humidity is preferably controlled to a water activity between 0.05 and 0.55, preferably between 0.1 and 0.5, depending on the type of biocatalyst enzyme system used. The reaction may be performed, for example, at 60°C in a stirred tank or in a packed bed reactor over biocatalysts, based on concentrates of Lipase D (*Rhizopus oryzae,* previously classified as *Rhizopus delemar,* from Amano Enzyme Inc., Japan) or immobilised concentrates of *Rhizomucor miehei* (Lipozyme RM IM from Novozymes A/S, Denmark).

In order to separate palmitic acid and other fatty acids or palmitic non-glyceride esters and other glycerides from OPO in (iv), the transesterified mixture (optionally after further treatment, such as isolation of the fat phase) is preferably distilled. Distillation is preferably carried out at low pressure (e.g., lower than 10 mbar) and elevated temperatures (e.g., greater than 200°C) to remove the fatty acids from the product triglyceride fraction.

The composition obtained in (iv) is preferably fractionated in (v) to recover an olein fraction (i.e., a lower melting fraction). This can be done using solvent fractionation, Lanza fractionation or dry fractionation, using a single, two-step or multi-step fractionation technique, but is preferably carried out using single step dry fractionation.

The olein can also be obtained by subjecting the transesterified mixture to multi-stage counter current dry fractionation.

Fractionation of the triglyceride fraction removes the unconverted tripalmitin (PPP) down to a level of less than 15 weight %, preferably less than 10 weight %, most preferably less than 8 weight %. The olein fraction, obtained after step (v), is typically further refined or purified to remove remaining fatty acids and contaminants to produce a refined olein fraction.

The process may optionally comprise further steps before, between or after (i) to (v), such as partial purification or enrichment of the products in the desired component(s).

The process may comprise an additional step of further purifying the product in OPO.

The composition obtained after (iv) or (v) preferably comprises at least 10% by weight OPO, more preferably at least 15%, even more preferably at least 20%, such as at least 25% or 30% or even 40% by weight OPO based on total glycerides in the fraction. The balance typically comprises other non-OPO triglycerides, and may further contain minor amounts of diglycerides and monoglycerides. Minor amounts of free fatty acids may also be present. The fraction is preferably a composition which comprises a mixture of triglycerides wherein different fatty acid residues, including unsaturated fatty acid residues, are randomly distributed between the 1- and 3-positions and at least half of the fatty acid residues in the 2-positions are C16 and/or C18 saturated, preferably consisting substantially of palmitic acid residues, in particular 60-90% by weight of the total 2-position fatty acids are preferably palmitic acid. Preferably, all of the fatty acid residues, or virtually all (e.g., greater than 99% by weight), in the glycerides of the composition are even-numbered. The unsaturated fatty acid residues in the 1- and 3-positions preferably consist largely of oleic acid and linoleic acid. The compositions preferably includes at least as much (on a molar basis) of saturated fatty acid in the 2-position as in the 1- and 3-positions combined, more preferably up to twice as much (on a molar basis). Preferably, the 1,3-positions include both unsaturated C18 and saturated C4 to C14 fatty acids. The proportion and type of these fatty acids may be determined in accordance with dietary and physical requirements of the composition required.

The composition obtained after (iv) or (v) is preferably blended with at least one vegetable oil to provide the composition for use in the invention. Typically, the amount of vegetable oil is from 40-70% by weight, based on the total weight of the fat blend.

Examples of suitable vegetable oils include sunflower oil, high oleic sunflower oil, palm kernel oil, rapeseed oil, flaxseed oil and mixtures thereof.

The composition of the invention is for use in developing and/or improving brain function, preferably for use in developing brain function in an infant human (typically from 0 to 4 years old). However, the invention may involve improving brain function in both infants and older humans. Brain function that may be developed and/or improved according to the invention includes one or more of: protection from neuroinflammation, modulation of the dopaminergic system, motor skills, intelligence (for example IQ), language skills and cognitive functions, including memory as well as attention, learning, reasoning, problem solving, and decision making. The invention may also involve the treatment and/or amelioration of addiction or of Alzheimer's disease.

In the invention, the glyceride having a palmitoyl group at the Sn-2 position increases OEA levels in the brain. Therefore, the invention also provides a method of increasing the level of OEA in the brain of a human by oral administration of the glyceride having a palmitoyl group at the 2- position and/or of the composition comprising the glyceride wherein at least 40% by weight of the palmitic acid content of the composition is present as palmitoyl groups bonded at the 2- position of a glyceride.

The composition of the invention is for oral administration, suitably as part of an otherwise normal diet.

A preferred product form for the composition of the invention is an infant formula.

An infant formula composition of the invention may comprise one or more components selected from other fats (i.e., lipids), protein, carbohydrate, minerals and vitamins. The present invention also therefore contemplates an infant formula comprising fat, protein and carbohydrate, for example in the approximate relative weight proportions of fat:protein:carbohydrate of 4-6:1-3:9-15. Dry formulations containing this mixture, together with additional components customary in such formulations may be dispersed in water to produce an emulsion of approximately 2 to 5 grams of fat per 100 ml of dispersion.

Alternatively, the composition may be in the form of a food product, food supplement or pharmaceutical product. The composition optionally comprises a complementary fat. For example, compositions in the form of food products may comprise a complementary fat selected from: cocoa butter, cocoa butter equivalents, palm oil or fractions thereof, palmkernel oil or fractions thereof, interesterified mixtures of said fats or fractions thereof, or liquid oils, selected from: sunflower oil, high oleic sunflower oil, soybean oil, rapeseed oil, cottonseed oil, fish oil, safflower oil, high oleic safflower oil, maize oil and MCT-oils. Food products (which term includes animal feed) contain a fat phase, wherein the fat phase contains the composition of the invention. The food products are suitably selected from the group consisting of: spreads, margarines, creams, dressings, mayonnaises, ice-creams, bakery products, infant food, chocolate, confectionery, sauces, coatings, cheese and soups. Food supplements or pharmaceutical products may be in the form of capsules or other forms, suitable for enteral or parenteral application and comprise a product of the invention.

The listing or discussion of an apparently prior-published document in this specification should not necessarily be taken as an acknowledgement that the document is part of the state of the art or is common general knowledge.

The following non-limiting examples illustrate the invention and do not limit its scope in any way. In the examples and throughout this specification, all percentages, parts and ratios are by weight unless indicated otherwise.

### EXAMPLES

### Example 1

60 male Wistar rats weight 100-150 g were housed for a week before allocating to 3 diets, control (regular chow for rodents), 10 % high Sn-2 diet (LD) and 10 % low Sn-2 tallow based diet (TD) (see Table 1 for fatty acid composition). Rats were fed for 5 weeks. Weight of the animals was recorded weekly, while food intake every 2 days. Length of the animals was also recorded weekly. Before sacrifice, rats were fasted for 12 h, and killed by decapitation. Brain tissue was taken and frozen at -80°C. Brain was analysed from 5 rats per group. Analyses of lipids included fatty acid profile of triglyceride (TAG) and phospholipid (PL) fraction. The endocannabinoid anandamide (AEA) and the PPAR alpha ligands oleoylethanolamide (OEA) and palmitoylethanolamide (PEA) were measured from total lipid extracts.

**Table 1**

| | High sn2 palmitic | Low sn2 palmitic |
|---|---|---|
| | LD | TD |
| IVFAME | 55 | 52 |
| PV mean | 1.5 | 1.7 |
| | | |
| Sn2 palmitic | 87.1 | 18.8 |
| | | |
| c14:0 | 1.3 | 3.4 |
| c16:0 | 23.5 | 24.2 |
| 16:1C | 2.1 | 3.4 |
| 16:1T | 0 | 0.4 |
| c17:0 | 0.4 | 1.2 |
| c18:0 | 21.3 | 19.1 |
| 18:1 total | 40.3 | 37.8 |
| 18:1T | 0.3 | 3.3 |
| 18:1C | 39.9 | 34.6 |
| 18:2 total | 8.9 | 8.3 |
| 18:2T | 0.2 | 1.5 |
| 18:2C | 8.7 | 6.8 |
| 18:3 total | 0.7 | 0.4 |
| 18:3C | 0.6 | 0.3 |
| 18:3T | 0.1 | 0.1 |

In the above table, IVFAME refers to calculated iodine value, PV is the peroxide value and Cx:y refers to a fatty acid having x carbon atoms and y double bonds. C refers to cis fatty acids and T to trans fatty acids.

### Results:

No changes were detected in PL and free fatty acid (FFA) fatty acid profiles in the brain.

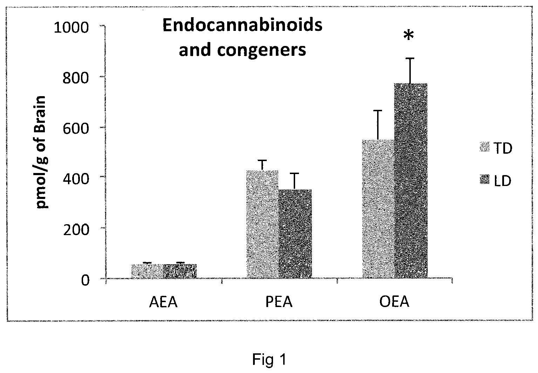

The results of AEA, PEA and OEA analysis are shown in Figure 1. The levels of AEA, PEA and OEA in the brain are shown for the low Sn-2 diet (TD; left hand bars in the figure) and the high Sn-2 diet (LD; right hand bars). There was a significant increase in the level of OEA.

This increase in OEA is expected to exert several beneficial effects, such as protection from neuroinflammation, modulation of the dopaminergic system and improvement of memory *via* the activation of PPAR alpha receptors.

It can be concluded that the position of palmitic acid in dietary triglycerides affects an increase of OEA biosynthesis, which may contribute in the physiological brain function and development, particularly in infants.

### Example 2

An infant formula is prepared comprising as a fat phase Betapol®45 (Loders Croklaan BV, Wormerveer, The Netherlands) being a fat composition having 47% by weight of the palmitic acid content of the composition present as palmitoyl groups bonded at the 2-position.

## Claims

1. 1,3-dioleoyl-2-palmitoyl glyceride (OPO) for use in developing and/or improving brain function, protecting against neuroinflammation or treating and/or ameliorating addiction or Alzheimer's disease.

2. OPO for use as claimed in claim 1 wherein the OPO is derived from a vegetable source.

3. OPO for use as claimed in any one of the preceding claims, wherein the OPO is produced by the reaction of palm oil stearin with oleic acid or an ester thereof in the presence of a lipase.

4. OPO for use as claimed in any one of the preceding claims, wherein the OPO is in the form of a composition which comprises alpha-linolenic acid or a glyceride thereof in an amount of greater than 1% by weight alpha-linolenic acid based on total free and bound C₆ to C₂₄ fatty acids and/or linoleic acid or a glyceride thereof, and wherein the weight ratio of total free and bound linoleic acid to total free and bound alpha-linolenic acid is preferably 15:1 or less.

5. OPO for use as claimed in any one of the preceding claims, wherein the OPO is in the form of a blend comprising (i) a composition comprising alpha-linolenic acid or a glyceride thereof and (ii) a triglyceride composition comprising OPO in an amount of at least 20% by weight, and wherein the composition comprising alpha-linolenic acid or a glyceride thereof is preferably a vegetable oil.

6. OPO for use as claimed in any one of the preceding claims, wherein the OPO is in the form of a composition which is free of phospholipids.

7. OPO for use as claimed in any one of the preceding claims, wherein the OPO is in the form of a composition which is an infant formula further comprising protein, carbohydrate, minerals and vitamins.

## Patentansprüche

1. 1,3-Dioleoyl-2-palmitoylglycerid (OPO) zur Verwendung beim Entwickeln und/oder Verbessern der Gehirnfunktion, Schützen gegen Neuroinflammation oder Behandeln und/oder Lindern von Sucht oder Alzheimer-Krankheit.

2. OPO zur Verwendung nach Anspruch 1, wobei das OPO von einer pflanzlichen Quelle stammt.

3. OPO zur Verwendung nach einem der vorhergehenden Ansprüche, wobei das OPO durch die Reaktion von Palmölstearin mit Ölsäure oder einem Ester davon in der Gegenwart einer Lipase hergestellt wird.

4. OPO zur Verwendung nach einem der vorhergehenden Ansprüche, wobei das OPO in der Form einer Zusammensetzung vorliegt, die Alpha-Linolensäure oder ein Glycerid davon in einer Menge von mehr als 1 Gew.-% Alpha-Linolensäure bezogen auf gesamte freie und gebundene C₆- bis C₂₄-Fettsäuren und/oder Linolensäure oder ein Glycerid davon umfasst, und wobei das Gewichtsverhältnis einer gesamten freien und gebundenen Linolensäure zu einer gesamten freien und gebundenen Alpha-Linolensäure vorzugsweise 15:1 oder weniger beträgt.

5. OPO zur Verwendung nach einem der vorhergehenden Ansprüche, wobei das OPO in der Form einer Mischung vorliegt, die Folgendes umfasst: (i) eine Zusammensetzung, umfassend Alpha-Linolensäure oder ein Glycerid davon, und (ii) eine Triglyceridzusammensetzung, umfassend OPO in einer Menge von wenigstens 20 Gew.-%, und wobei die Zusammensetzung, umfassend Alpha-Linolensäure oder ein Glycerid davon, vorzugsweise ein pflanzliches Öl ist.

6. OPO zur Verwendung nach einem der vorhergehenden Ansprüche, wobei das OPO in der Form einer Zusammensetzung vorliegt, die frei von Phospholipiden ist.

7. OPO zur Verwendung nach einem der vorhergehenden Ansprüche, wobei das OPO in der Form einer Zusammensetzung vorliegt, die eine Säuglingsanfangsnahrung ist, die ferner Protein, Kohlenhydrate, Mineralien und Vitamine umfasst.

## Revendications

1. 1,3-dioléoyl-2-palmitoyl glycéride (OPO) à utiliser dans le développement et/ou l'amélioration de la fonction cérébrale, dans la protection contre la neuroinflammation ou dans le traitement et/ou l'amélioration d'addiction ou de la maladie d'Alzheimer.

2. OPO à utiliser selon la revendication 1 dans lequel l'OPO est dérivé d'une source végétale.

3. OPO à utiliser selon l'une quelconque des revendications précédentes, l'OPO étant produit par la réaction de stéarine d'huile de palme avec de l'acide oléique, ou un ester de celui-ci, en présence d'une lipase.

4. OPO à utiliser selon l'une quelconque des revendications précédentes, l'OPO étant sous la forme d'une composition qui contient de l'acide alpha-linolénique, ou un glycéride de celui-ci, dans une quantité supérieure à 1 % en poids d'acide alpha-linolénique par rapport aux acides gras en C₆-C₂₄ et/ou à l'acide linoléique, ou un glycéride de celui-ci, libres et liés totaux, et dans lequel le rapport pondéral entre l'acide linoléique libre et lié total et l'acide alpha-linolénique libre et lié total est de préférence de 15:1 ou inférieur.

5. OPO à utiliser selon l'une quelconque des revendications précédentes, l'OPO étant sous la forme d'un mélange comprenant (i) une composition contenant de l'acide alpha-linolénique, ou un glycéride de celui-ci, et (ii) une composition de triglycéride comprenant l'OPO dans une quantité d'au moins 20 % en poids, et la composition contenant de l'acide alpha-linolénique, ou un glycéride de celui-ci, étant de préférence une huile végétale.

6. OPO à utiliser selon l'une quelconque des revendications précédentes, l'OPO étant sous la forme d'une composition qui est exempte de phospholipides.

7. OPO à utiliser selon l'une quelconque des revendications précédentes, l'OPO étant sous la forme d'une composition qui est une préparation pour nourrissons contenant en outre une protéine, un glucide, des minéraux et des vitamines.
